# EUROPEAN PATENT APPLICATION

(11) **EP 3 770 254 A1**
(43) Date of publication of application: **27.01.2021**
(21) Application number: 19771166.6
(22) Date of filing: 22.03.2019
(51) Int. Cl.: C12N 9/96, A23L 7/104, A23L 13/00, A23L 17/00, C12N 9/10

(54) **LIQUID PREPARATION CONTAINING TRANSGLUTAMINASE**

(30) Priority: 23.03.2018 JP 2018057185
(71) Applicant: AJINOMOTO CO., INC., Chuo-ku Tokyo 104-8315 (JP)
(72) Inventor: OHIRA, Takuya, Kawasaki-shi, Kanagawa 210-8681 (JP); YOKOYAMA, Noriko, Kawasaki-shi, Kanagawa 210-8681 (JP)
(74) Representative: Strehl Schübel-Hopf & Partner
(86) International application number: PCT/JP2019/012142
(87) International publication number: WO 2019/182123

(57) **Abstract**

The present invention provides a liquid preparation comprising transglutaminase, and one or more kinds selected from the group consisting of glycine, proline, serine, a glutamic acid salt, an aspartic acid salt and an organic acid salt, wherein a total content of said one or more kinds selected from the group consisting of glycine, proline, serine, a glutamic acid salt, an aspartic acid salt and an organic acid salt is not less than 2 wt% or not less than 5 wt%, that shows improved stability of transglutaminase and is easily utilized in the field of food.

## Description

### [Technical Field]

The present invention relates to a liquid preparation containing transglutaminase. More particularly, the present invention relates to a liquid preparation with improved stability of transglutaminase.

### [Background Art]

Transglutaminase is an enzyme that condenses an amino group of a glutamine residue in a protein with a primary amine, transfers a substituent on the amine to a glutamine residue, and catalyzes crosslinking of the protein. It is widely used for modification, processing and the like of meat. From the aspect of the stability of enzyme, a preparation containing transglutaminase is provided as a solid preparation such as powder, granule or the like.

When transglutaminase is acted on a substrate, it is often used as a liquid preparation obtained by dissolving in an appropriate solvent. Since conventional solid preparations require dissolution in a solvent when in use, they lack convenience and are further associated with a problem of contamination with microorganism when dissolving in a solvent.

As a liquid preparation containing transglutaminase, a preparation containing 25 wt% - 100 wt% of polyol, in which transglutaminase is contained in a polyol-water suspension with a pH value within the range of 4.4 - 5.1 (patent document 1), and a preparation in which transglutaminase is dissolved together with a water activity adjuster, an oxidation reduction electric potential regulator, a preservative and a pH adjuster (patent document 2) are disclosed.

However, the preparation described in patent document 1 requires control of the pH of the polyol-water suspension to a very narrow range of 4.4 - 5.1. The preparation described in patent document 2 requires blending of considerable kinds of stabilizers to stabilize transglutaminase, which may limit use of the enzyme preparation.

On the other hand, use of sugar, sugar alcohol or amino acid as a stabilizer of a lyophilized preparation of transglutaminase is disclosed (patent documents 3, 4).

However, the techniques described in patent documents 3 and 4 relate to a lyophilized preparation of a blood coagulation factor XIII, transglutaminase, and do not aim to stabilize transglutaminase, which is used in the field of food, in a liquid preparation.

Thus, a liquid preparation of transglutaminase that is easily used in the field of food and has high stability has been desired.

### [Document List]

### [Patent documents]

patent document 1: National Publication of International Patent Application No. 2014-532421
patent document 2: CN 105462950
patent document 3: JP-B-3530300
patent document 4: JP-B-6244079

### [Summary of Invention]

### [Technical Problem]

The present invention aims to provide a liquid preparation containing transglutaminase, that shows improved stability of transglutaminase and is easily utilized in the field of food.

### [Solution to Problem]

The present inventors have conducted intensive studies in an attempt to solve the above-mentioned problems and found that a liquid preparation containing transglutaminase, and one or more kinds selected from the group consisting of glycine, proline, serine, a glutamic acid salt, an aspartic acid salt and an organic acid salt at a concentration of not less than 2 wt% or not less than 5 wt% as a total content of these shows improved stability of transglutaminase in the liquid preparation, which resulted in the completion of the present invention.

That is, the present invention relates to the following.
[1] A liquid preparation comprising transglutaminase, and one or more kinds selected from the group consisting of glycine, proline, serine, a glutamic acid salt, an aspartic acid salt and an organic acid salt, wherein a total content of said one or more kinds selected from the group consisting of glycine, proline, serine, a glutamic acid salt, an aspartic acid salt and an organic acid salt is not less than 5 wt%.
[2] The liquid preparation of [1], wherein the transglutaminase is derived from a microorganism.
[3] The liquid preparation of [1] or [2], wherein the total content of said one or more kinds selected from the group consisting of glycine, proline, serine, a glutamic acid salt, an aspartic acid salt and an organic acid salt is not less than 10 wt%.
[4] The liquid preparation of any of [1] to [3], wherein the organic acid salt is one or more kinds selected from the group consisting of an acetic acid salt, a citric acid salt and a gluconic acid salt.
[5] The liquid preparation of any of [1] to [4], wherein the glutamic acid salt, aspartic acid salt and organic acid salt are each a sodium salt.
[6] The liquid preparation of any of [1] to [5], wherein the liquid preparation has pH 4 - 7.
[7] A liquid preparation comprising transglutaminase, and one or more kinds selected from the group consisting of glycine, proline, serine, a glutamic acid salt, an aspartic acid salt and an organic acid salt, wherein a total content of said one or more kinds selected from the group consisting of glycine, proline, serine, a glutamic acid salt, an aspartic acid salt and an organic acid salt is not less than 2 wt%.
[8] A method for producing a liquid preparation comprising dissolving transglutaminase, and one or more kinds selected from the group consisting of glycine, proline, serine, a glutamic acid salt, an aspartic acid salt and an organic acid salt by adding them to a solvent, wherein a total content of said one or more kinds selected from the group consisting of glycine, proline, serine, a glutamic acid salt, an aspartic acid salt and an organic acid salt is not less than 5 wt%.
[9] The production method of [8], wherein the transglutaminase is derived from a microorganism.
[10] The production method of [8] or [9], wherein the total content of said one or more kinds selected from the group consisting of glycine, proline, serine, a glutamic acid salt, an aspartic acid salt and an organic acid salt is not less than 10 wt%.
[11] The production method of any of [8] to [10], wherein the organic acid salt is one or more kinds selected from the group consisting of an acetic acid salt, a citric acid salt and a gluconic acid salt.
[12] The production method of any of [8] to [11], wherein the glutamic acid salt, aspartic acid salt and organic acid salt are each a sodium salt.
[13] The production method of any of [8] to [12], comprising setting the pH of the liquid preparation to 4 - 7.
[14] A method for producing a liquid preparation comprising dissolving transglutaminase, and one or more kinds selected from the group consisting of glycine, proline, serine, a glutamic acid salt, an aspartic acid salt and an organic acid salt by adding them to a solvent, wherein a total content of said one or more kinds selected from the group consisting of glycine, proline, serine, a glutamic acid salt, an aspartic acid salt and an organic acid salt is not less than 2 wt%.
[15] A method for stabilizing transglutaminase in a liquid preparation, comprising adding one or more kinds selected from the group consisting of glycine, proline, serine, a glutamic acid salt, an aspartic acid salt and an organic acid salt to a liquid preparation comprising transglutaminase such that a total content of these is not less than 5 wt%.
[16] The stabilizing method of [15], wherein the transglutaminase is derived from a microorganism.
[17] The stabilizing method of [15] or [16], wherein the total content of said one or more kinds selected from the group consisting of glycine, proline, serine, a glutamic acid salt, an aspartic acid salt and an organic acid salt is not less than 10 wt%.
[18] The stabilizing method of any of [15] to [17], wherein the organic acid salt is one or more kinds selected from the group consisting of an acetic acid salt, a citric acid salt and a gluconic acid salt.
[19] The stabilizing method of any of [15] to [18], wherein the glutamic acid salt, aspartic acid salt and organic acid salt are each a sodium salt.
[20] The stabilizing method of any of [15] to [19], comprising setting the pH of the liquid preparation to 4 - 7.
[21] A method for stabilizing transglutaminase in a liquid preparation, comprising adding one or more kinds selected from the group consisting of glycine, proline, serine, a glutamic acid salt, an aspartic acid salt and an organic acid salt to a liquid preparation comprising transglutaminase such that a total content of these is not less than 2 wt%.
[22] A food comprising the liquid preparation of any of [1] to [6] .
[23] A food comprising the liquid preparation of [7].

### [Advantageous Effects of Invention]

According to the present invention, a liquid preparation of transglutaminase showing improved stability of transglutaminase can be provided.

The liquid preparation of the present invention does not require dissolution of transglutaminase in a solvent when in use, is highly convenient, and is associated with less risk of microorganism contamination and the like. It is suitable for use in the field of food.

### [Brief Description of Drawings]

Fig. 1 shows the residual rate of the transglutaminase activity of the liquid preparations of Examples 1 - 5 and Comparative Example 1 in Experimental Example 1.
Fig. 2 shows the residual rate of the transglutaminase activity of the liquid preparations of Examples 1-1 to 1-3 and Comparative Example 2 in Experimental Example 2(1).
Fig. 3 shows the residual rate of the transglutaminase activity of the liquid preparations of Examples 2-1 to 2-4 and Comparative Example 3 in Experimental Example 2(2).
Fig. 4 shows the residual rate of the transglutaminase activity of the liquid preparations of Examples 3-1 to 3-3 and Comparative Example 4 in Experimental Example 2(3).
Fig. 5 shows the residual rate of the transglutaminase activity of the liquid preparations of Examples 4-1 to 4-4 and Comparative Example 5 in Experimental Example 2(4).
Fig. 6 shows the relationship between the pH and transglutaminase activity of the liquid preparations in a glycine addition section and a glycine non-addition section in Experimental Example 3.
Fig. 7 shows the residual rate of the transglutaminase activity of the liquid preparations of Example 6 and Comparative Examples 6-1, 6-2 in Experimental Example 4.
Fig. 8 shows the residual rate of the transglutaminase activity of the liquid preparations of Example 7 and Comparative Examples 7-1, 7-2 in Experimental Example 4.
Fig. 9 shows the residual rate of the transglutaminase activity of the liquid preparations of Example 8 and Comparative Examples 8-1, 8-2 in Experimental Example 4.

### [Description of Embodiments]

The present invention provides a liquid preparation containing transglutaminase and having improved stability of transglutaminase (hereinafter to be also referred to as "the preparation of the present invention" in the present specification".

The preparation of the present invention contains, together with transglutaminase, one or more kinds selected from the group consisting of glycine, proline, serine, a glutamic acid salt, an aspartic acid salt and an organic acid salt at a concentration of not less than 5 wt%.

In another embodiment, the preparation of the present invention contains, together with transglutaminase, one or more kinds selected from the group consisting of glycine, proline, serine, a glutamic acid salt, an aspartic acid salt and an organic acid salt at a concentration of not less than 2 wt%.

Transglutaminase (protein-glutamine γ-glutamyl transferase) is a transferase that catalyzes a reaction to form ammonia by condensing an amino group of a glutamine residue in a protein with a primary amine, and transferring a substituent on the amine to a glutamine residue. Generally, an amino group of a lysine residue in a protein is used as the primary amine, and transglutaminase acts as a crosslinking enzyme.

Therefore, transglutaminase is preferably used for modification, processing and the like of meat such as fish meat, livestock meat and the like.

As transglutaminase, calcium independent transglutaminase obtained from a microorganism is preferably used.

As the calcium independent transglutaminase derived from a microorganism, transglutaminase produced by actinomycetes belonging to streptomyces can be mentioned, and it can be obtained according the method described in JP-B-2572716 and the like. Commercially available products such as "Activa TG-K" and "Activa TG-S" provided by Ajinomoto Co., Inc. and the like can also be used.

The content of transglutaminase in the preparation of the present invention is preferably 1 U (unit) to 1,000 U, more preferably 10 U to 350 U, per 1 g of the preparation of the present invention.

The enzyme activity of transglutaminase can be measured and calculated by, for example, the hydroxamate method. That is, benzyloxycarbonyl-L-glutaminyl glycine is reacted with hydroxylamine as a substrate to form an iron complex of hydroxamic acid formed in the aforementioned reaction in the presence of trichloroacetic acid, the absorbance at 525 nm is measured, and the amount of hydroxamic acid produced is determined from the calibration curve, whereby the enzyme activity can be calculated. In the present specification, the amount of enzyme that produces 1 µmol of hydroxamic acid in 1 min at 37°C, pH=6.0 is defined as 1 U (see JP-A-s64-027471).

In the preparation of the present invention, glycine contained together with transglutaminase is 2-aminoacetic acid, which has the simplest structure among amino acids constituting proteins and is classified as a non-polar side chain amino acid.

Proline is a pyrrolidine-2-carboxylic acid, which is a cyclic amino acid.

Serine is 2-amino-3-hydroxypropionic acid, which is a hydroxyamino acid classified as a polar uncharged side chain amino acid.

The preparation of the present invention contains glycine, proline and serine each in a free form.

Glutamic acid salt is a salt of 2-aminopentanedioic acid classified as an acidic polar side chain amino acid.

Aspartic acid salt is a salt of 2-aminobutanedioic acid classified as an acidic polar side chain amino acid.

Proline, serine, glutamic acid salt and aspartic acid salt may be used in any of L-form, D-form and DL-form. L-form and DL-form are preferable, and L-form is further preferable.

In the preparation of the present invention, examples of the glutamic acid salt and aspartic acid salt specifically include salts with inorganic base, organic base, inorganic acid, organic acid and salts with amino acid and the like.

Examples of the salt with inorganic base include salts with alkali metals such as lithium, sodium, potassium and the like, salts with alkaline earth metals such as magnesium, calcium and the like, ammonium salt and the like.

Examples of the salt with organic base include salts with alkanol amines such as monoethanolamine, diethanolamine, triethanolamine and the like, salts with heterocyclic amines such as morpholine, piperidine and the like, and the like.

Examples of the salt with inorganic acid include salts with hydrohalic acid (hydrochloric acid, hydrobromic acid, hydroiodic acid etc.), sulfuric acid, nitric acid, phosphoric acid and the like.

Examples of the salt with organic acid include salts with monocarboxylic acids such as formic acid, acetic acid, propanoic acid and the like; salts with saturated dicarboxylic acids such as oxalic acid, malonic acid, malic acid, succinic acid and the like; salts with unsaturated dicarboxylic acids such as maleic acid, fumaric acid and the like; salts with tricarboxylic acids such as citric acid and the like; and salts with keto acids such as α-ketoglutar acid and the like, and the like.

Examples of the salt with amino acid include salts with aliphatic amino acids such as glycine, alanine and the like; salts with aromatic amino acids such as phenylalanine and the like; salts with basic amino acids such as lysine and the like; salts with amino acids that form lactam such as pyroglutamic acid and the like, and the like.

From the aspects of solubility and the transglutaminase stabilizing effect of the preparation of the present invention, alkali metal salts such as sodium salt and the like are preferably used as the glutamic acid salt and aspartic acid salt.

In the preparation of the present invention, not only normal salts but also acidic salts (hydrogen salts) are preferably used as the above-mentioned glutamic acid salt and aspartic acid salt, and hydrates thereof can also be used.

When these hydrates are used as the glutamic acid salt and aspartic acid salt, the contents of the glutamic acid salt and aspartic acid salt in the preparation of the present invention are each shown by a content converted to that of anhydrate.

In the present invention, as the above-mentioned glycine, proline, serine, glutamic acid salt and aspartic acid salt, any of those obtained by extraction and purification from naturally occurring plants and animals, or those obtained by chemical synthesis method, fermentation method, enzyme method, gene recombination method, or the like may be used. Commercially available products provided by various companies may also be used.

In the preparation of the present invention, the organic acid salt contained together with transglutaminase is not particularly limited as long as it is a salt of an acid of an organic compound, can be dissolved in a liquid preparation, is edible, and can be utilized for food. For example, salts of saturated monocarboxylic acids such as formic acid, acetic acid, propanoic acid and the like; salts of unsaturated monocarboxylic acids such as sorbic acid and the like; salts of hydroxymonocarboxylic acids such as glycolic acid, lactic acid, glycerol acid and the like; salts of saturated dicarboxylic acids such as oxalic acid, malonic acid, succinic acid and the like; salts of unsaturated dicarboxylic acids such as maleic acid, fumaric acid and the like; salts of hydroxydicarboxylic acids such as malic acid, tartaric acid and the like; salts of hydroxytricarboxylic acids such as citric acid, isocitric acid and the like; salts of keto acids such as pyruvic acid, oxaloacetic acid, α-ketoglutar acid and the like, salts of aldonic acids having about 5 - 6 carbon atoms such as gluconic acid, galactonic acid, mannoic acid and the like; salts of aldaric acids having about 5 - 6 carbon atoms such as glucaric acid, galactaric acid, mannaric acid and the like; and salts of uronic acids having about 5 - 6 carbon atoms such as fructuronic acid, glucuronic acid, galacturonic acid, mannuronic acid and the like are preferably used, and acetic acid salt, citric acid salt, and gluconic acid salt are more preferably used.

Acetic acid salt, citric acid salt and the like are also preferable in that they function as a buffering agent described later.

Examples of the salts of the above-mentioned organic acids include salts with inorganic bases such as salts with alkali metals (e.g. lithium, sodium, potassium and the like), salts with alkaline earth metals (e.g. magnesium, calcium and the like), ammonium salt and the like; salts with organic bases such as salts with alkanol amines (e.g. monoethanolamine, diethanolamine, triethanolamine and the like), salts with heterocyclic amines (e.g. morpholine, piperidine and the like) and the like. Alkali metal salts are preferably used, and a sodium salt is more preferably used.

In the preparation of the present invention, not only normal salts but also acid salts (hydrogen salt) are preferably used as the above-mentioned organic acid salt, and hydrates of these can also be used.

When hydrate is used as the organic acid salt, the content of the organic acid salt in the preparation of the present invention is shown by a content converted to that of anhydrate.

In the present invention, as the above-mentioned organic acid salt, any of those obtained by extraction and purification from naturally occurring plants and animals, or those obtained by chemical synthesis method, fermentation method, enzyme method, gene recombination method, or the like may be used. Commercially available products provided by various companies may also be used.

In the preparation of the present invention, one or more kinds selected from the group consisting of the above-mentioned glycine, proline, serine, glutamic acid salt, aspartic acid salt and organic acid salt can be used.

The preparation of the present invention contains not less than 5 wt%, preferably not less than 10 wt%, of one or more kinds selected from the group consisting of glycine, proline, serine, a glutamic acid salt, an aspartic acid salt and an organic acid salt at a total content of these.

In another embodiment of the present invention, the preparation of the present invention contains not less than 2 wt% of one or more kinds selected from the group consisting of glycine, proline, serine, a glutamic acid salt, an aspartic acid salt and an organic acid salt as a total content of these.

On the other hand, the total content of one or more kinds selected from the group consisting of glycine, proline, serine, a glutamic acid salt, an aspartic acid salt and an organic acid salt in the preparation of the present invention is generally not more than 20 wt%. A total content of the aforementioned amino acid and the like exceeding 20 wt% is not economical because the stabilizing effect of transglutaminase reaches a plateau and the effect commensurate with the content of amino acid and the like cannot be expected.

The preparation of the present invention is a liquid preparation containing transglutaminase, and one or more kinds selected from the group consisting of glycine, proline, serine, a glutamic acid salt, an aspartic acid salt and an organic acid salt dissolved in a solvent.

As used herein, the "liquid preparation" refers to a preparation in the form of a solution at room temperature, and includes a preparation in the form of a liquid having viscosity.

The "room temperature" means a room temperature defined in the Seventeenth Edition of the Japanese Pharmacopoeia, General Rules, that is, 1°C to 30°C.

As the solvent for dissolving transglutaminase and the above-mentioned amino acids, and the like, water suitable for food production such as purified water, deionized water, tap water or the like is preferably used. In addition, as described later, a buffer such as acetate buffer, phosphate buffer and the like can also be used as a solvent.

In the present invention, from the aspect of the stability of transglutaminase, the pH of the preparation of the present invention is preferably 4 - 7, more preferably 4 - 6, further preferably 5 - 6.

The pH of the preparation of the present invention is measured by a general glass electrode method at 20°C.

The pH of the preparation of the present invention can be adjusted using a pH adjuster or buffering agent. The pH adjuster or buffering agent is not particularly limited as long as it can adjust the pH of a solution containing transglutaminase, the above-mentioned amino acids and the like to a desired range, and is edible. Examples thereof include hydrochloric acid, citric acid, sodium citrate, succinic acid, acetic acid, sodium acetate, potassium hydroxide, sodium hydroxide, sodium hydrogen carbonate, sodium carbonate, lactic acid, sodium lactate, phosphoric acid, trisodium phosphate, sodium hydrogenphosphate, sodium dihydrogen phosphate and the like.

Among these, citric acid, sodium citrate, acetic acid, sodium acetate, phosphoric acid, sodium hydrogenphosphate, sodium dihydrogen phosphate and the like are preferably used.

The preparation of the present invention can also be produced using citrate buffer, acetate buffer, phosphate buffer or the like adjusted to pH 4 - 7 as the solvent. The concentration of various buffering agents in the buffer is preferably set to about 0.01 M - 1 M.

Furthermore, the preparation of the present invention can contain food additives such as thickening stabilizer (sodium alginate, xanthan gum, carboxymethylcellulose sodium etc.), preservative (sodium benzoate, sodium edetate, potassium sorbate etc.), antioxidant (ascorbic acid, erythorbic acid etc.) and the like as long as the characteristics of the present invention are not impaired.

The preparation of the present invention can be produced by adding transglutaminase, and one or more kinds selected from the group consisting of glycine, proline, serine, a glutamic acid salt, an aspartic acid salt and an organic acid salt together with pH adjuster or buffering agent, and other food additives as necessary to a solvent such as water and the like, followed by mixing and dissolving.

In addition, the preparation of the present invention can also be produced by adding transglutaminase, and one or more kinds selected from the group consisting of glycine, proline, serine, a glutamic acid salt, an aspartic acid salt and an organic acid salt together with other food additives as necessary to a buffer preferably adjusted to pH 4 - 7, followed by mixing and dissolving.

In the preparation of the present invention, the stability of transglutaminase is improved, and the preparation can be preserved for a long time under general storage conditions (e.g., refrigerated storage etc.).

Therefore, it is highly convenient because it does not require dissolution in a solvent when in use, and the risk of microbial contamination and the like during the operation of dissolving in a solvent is also reduced.

The present invention also provides a method for producing a stable liquid preparation containing transglutaminase (hereinafter to be also referred to as "the production method of the present invention" in the present specification).

The production method of the present invention includes dissolving transglutaminase, and one or more kinds selected from the group consisting of glycine, proline, serine, a glutamic acid salt, an aspartic acid salt and an organic acid salt by adding them to a solvent.

In the production method of the present invention, the total content of one or more kinds selected from the group consisting of glycine, proline, serine, a glutamic acid salt, an aspartic acid salt and an organic acid salt in the liquid preparation is not less than 5 wt%, preferably not less than 10 wt%.

In another embodiment of the production method of the present invention, the total content of one or more kinds selected from the group consisting of glycine, proline, serine, a glutamic acid salt, an aspartic acid salt and an organic acid salt in a liquid preparation is not less than 2 wt%.

On the other hand, the total content of one or more kinds selected from the group consisting of glycine, proline, serine, a glutamic acid salt, an aspartic acid salt and an organic acid salt in a liquid preparation is generally not more than 20 wt% because the stabilizing effect of transglutaminase reaches a plateau when the total content of the above-mentioned amino acid and the like exceeds 20 wt%.

Transglutaminase, glycine, proline, serine, glutamic acid salt, aspartic acid salt and organic acid salt, and the solvent that dissolves these are as described above for the preparation of the present invention.

In the production method of the present invention, the pH of the liquid preparation is controlled to preferably 4 - 7, more preferably 4 - 6, further preferably 5 - 6, using a pH adjuster or a buffering agent, or using a buffer as a solvent.

The pH adjuster or buffering agent and buffer, and the measurement method of pH are as described above for the preparation of the present invention.

In the production method of the present invention, food additives other than pH adjuster or buffering agent, for example, thickening stabilizer, preservative, antioxidant and the like can also be added as long as the characteristics of the present invention are not impaired.

Such other food additives are also as described above.

The production method of the present invention can provide a liquid preparation in which transglutaminase is stabilized, which preparation can be preserved for a long time under general storage conditions (e.g., refrigerated storage etc.), does not require dissolution in a solvent when in use, and is highly convenient.

Furthermore, the present invention provides a method for stabilizing transglutaminase in a liquid preparation (hereinafter to be also referred to as "the stabilizing method of the present invention" in the present specification).

The stabilizing method of the present invention includes adding one or more kinds selected from the group consisting of glycine, proline, serine, a glutamic acid salt, an aspartic acid salt and an organic acid salt to a liquid preparation comprising transglutaminase such that a total content of these is not less than 5 wt%.

In the stabilizing method of the present invention, the total content of one or more kinds selected from the group consisting of glycine, proline, serine, a glutamic acid salt, an aspartic acid salt and an organic acid salt is preferably not less than 10 wt%.

In another embodiment of the stabilizing method of the present invention, the total content of one or more kinds selected from the group consisting of glycine, proline, serine, a glutamic acid salt, an aspartic acid salt and an organic acid salt is not less than 2 wt%.

The total content of one or more kinds selected from the group consisting of glycine, proline, serine, a glutamic acid salt, an aspartic acid salt and an organic acid salt in a liquid preparation is generally not more than 20 wt% because the stabilizing effect of transglutaminase reaches a plateau when the total content of the above-mentioned amino acid and the like exceeds 20 wt%.

Transglutaminase, glycine, proline, serine, glutamic acid salt, aspartic acid salt and organic acid salt, and the solvent used for the liquid preparation are as described above for the preparation of the present invention.

In the stabilizing method of the present invention, the pH of the a liquid preparation containing transglutaminase is controlled to preferably 4 - 7, more preferably 4 - 6, further preferably 5 - 6, using a pH adjuster or a buffering agent, or using a buffer as a solvent.

The pH adjuster or buffering agent and buffer, and the measurement method of pH are as described above for the preparation of the present invention.

In the stabilizing method of the present invention, food additives other than pH adjuster or buffering agent, for example, thickening stabilizer, preservative, antioxidant and the like can also be added as long as the characteristics of the present invention are not impaired.

Such other food additives are also as described above for the preparation of the present invention.

The stabilizing method of the present invention can provide a liquid preparation in which transglutaminase in the liquid preparation is stabilized, which preparation can be preserved for a long time under general storage conditions (e.g., refrigerated storage etc.), does not require dissolution in a solvent when in use, and is highly convenient.

Furthermore, the present invention provides a food added with the preparation of the present invention (hereinafter to be also referred to as "the food of the present invention" in the present specification).

Preferable examples of the food of the present invention include, but are not limited to, meat processed foods (e.g., processed product of salted meat such as ham, bacon and the like; meat processed paste products such as sausage, hamburg, meat ball, shumai, gyoza, meat bun, grilled chicken meat ball, fried cake of minced meat and the like, etc.), processed seafood products (e.g., fish meat sausage, boiled fish paste, chikuwa, satsuma-age, hanpen, fish ball, shrimp ball etc.), rice foods (e.g., cooked polished rice, rice boiled with red beans, pilaf, rice seasoned and cooked with various ingredients, rice gruel, risotto, rice ball, sushi, box lunch, rice noodle etc.), noodles (e.g., noodles such as Japanese wheat noodles, pasta, Japanese soba, Chinese noodles, fried noodles, instant noodles that go through flying step, drying step and the like, sheet for gyoza or shumai, etc.), grain flour (e.g., wheat flour, barley flour, corn flour, buckwheat flour, rye flour, oat flour, millet flour, pea flour, soy flour etc.) and processed foods thereof, dairy products (yogurt, cheese, cream, butter, butter oil, cheese, concentrated whey, various ice creams, concentrated milk, condensed milk, cream powder, whey powder, butter milk powder, milk powder, fermented milk, lactic fermenting beverage, milk beverage etc.), various tofu (e.g., silken tofu, firm tofu, soft tofu, filled tofu etc.) and processed products thereof (e.g., fried tofu, fried silken tofu, broiled tofu, fried tofu (thick deep-fried bean curd, deep-fried tofu, deep-fried tofu for sushi etc.); tofu processed paste products (deep-fried tofu mixed with thinly sliced vegetables, tofu tube-shaped fish sausage, tofu fish cake etc.) using soy milk, tofu dough, separation soybean protein and the like as starting materials; freeze-dried tofu; and soy milk-tofu desserts (soy milk purine, soy milk jelly, soy milk yogurt etc.)).

The amount of the preparation of the present invention to be added to the food of the present invention can be appropriately determined depending on the form of the food of the present invention, the kind of the starting materials contained in the food, the processing means, and the like. It is preferably added such that the titer of transglutaminase per 1 g of food is 0.00001U - 1000U, more preferably 0.0001U - 100U, further preferably 0.001U - 10U.

The food of the present invention can be produced by adding general food additives as necessary to the food materials such as meat and the like and the preparation of the present invention, and according to a general food production method.

The present invention can provide a food in which stabilized transglutaminase acts on food materials such as meat and the like to improve mouthfeel.

The present invention is explained in further detail by the following Examples.

### [Examples 1 - 5, Comparative Example 1] liquid preparation containing transglutaminase

Microorganism-derived transglutaminase (1,000U/g, "ActivaTG", Ajinomoto Co., Inc.) (17.6 wt%) and glycine, proline, serine, sodium glutamate and sodium aspartate (each 10 wt%) were dissolved in 0.05 M phosphate buffer (pH=6.0) (72.4 wt%) to give the liquid preparations of Examples 1 - 5.

On the other hand, the same amount of the above-mentioned transglutaminase was dissolved in 0.05 M phosphate buffer (pH=6.0, 72.4 wt%) together with lysine hydrochloride (10 wt%) to give the liquid preparation of Comparative Example 1.

### [Experimental Example 1] Evaluation of stabilizing effect of transglutaminase

The stability of transglutaminase in each liquid preparation of the above-mentioned Examples 1 - 5 and Comparative Example 1 was evaluated by an accelerated test by high temperature storage as follows.

Respective liquid preparations of the above-mentioned Examples 1 - 5 and Comparative Example 1 were stored at 4°C and 44°C for 24 hr, and the transglutaminase activity in the liquid preparation was measured by the above-mentioned hydroxamate method.

A liquid preparation obtained by dissolving only transglutaminase in a phosphate buffer was used as a control and treated in the same manner.

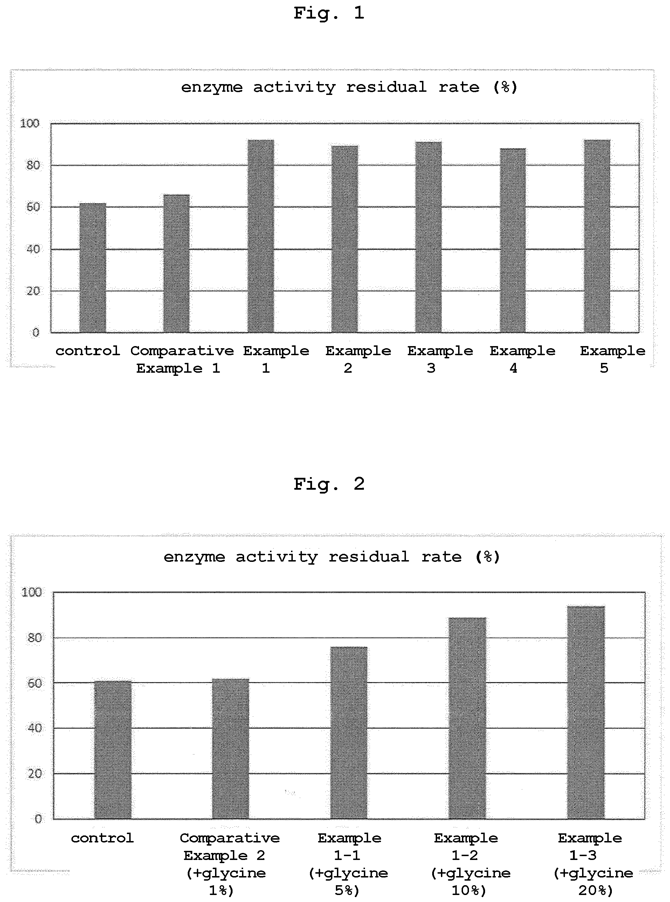

The stability of transglutaminase in each liquid preparation is shown in Table 1 and Fig. 1 by the residual rate (%) of the transglutaminase activity when stored at 44°C with respect to the transglutaminase activity when stored at 4°C.

**[Table 1]**

| sample | amino acid contained | activity residual rate (%) |
|---|---|---|
| control | - | 62 |
| Comparative Example 1 | lysine hydrochloride | 66 |
| Example 1 | glycine | 92 |
| Example 2 | proline | 89 |
| Example 3 | serine | 91 |
| Example 4 | sodium glutamate | 88 |
| Example 5 | sodium aspartate | 92 |

| | | |
|---|---|---|
| *; In the table, "-" means that amino acid or amino acid salt is not contained. | | |

As shown in Table 1 and Fig. 1, the transglutaminase activity residual rate of the control was 62%.

In contrast, the liquid preparations of Examples 1 - 5 of the present invention showed a high transglutaminase activity residual rate of around 90%.

On the other hand, in the liquid preparation of Comparative Example 1, the residual activity rate of transglutaminase was 66%, which was similar to that of the control.

From the above-mentioned results of Experimental Example 1, it was confirmed that each of glycine, proline, serine, sodium glutamate and sodium aspartate has a transglutaminase stabilizing effect in a liquid preparation.

On the other hand, a transglutaminase stabilizing effect was not found in lysine hydrochloride which is a salt of amino acid other than the above-mentioned amino acids.

### [Experimental Example 2] Study of influence of content of amino acid or amino acid salt on transglutaminase stabilizing effect

Influence of the content of amino acid or amino acid salt in a liquid preparation on the stability of transglutaminase was evaluated by an accelerated test by high temperature storage as follows.

### (1) Glycine content

Glycine was added to and dissolved at 5 wt%, 10 wt% and 20 wt% in 0.05 M phosphate buffer (pH=6.0) containing microorganism-derived transglutaminase (1,000U/g, "ActivaTG", Ajinomoto Co., Inc.) (17.6 wt%), and sodium benzoate (0.1 wt%). 0.05 M Phosphate buffer (pH=6.0) was used to make the total amount 100 wt% and the liquid preparations were used as Examples 1-1, 1-2 and 1-3, respectively.

In addition, a liquid preparation prepared similarly with a glycine content of 1 wt% was used as Comparative Example 2.

Respective liquid preparations of Examples 1-1 to 1-3 and Comparative Example 2 were stored at 4°C and 44°C for 24 hr, and the transglutaminase activity in the liquid preparation was measured by the above-mentioned hydroxamate method. A liquid preparation similarly produced without adding glycine was used as a control and treated in the same manner.

The transglutaminase activity when stored at 44°C is shown in Fig. 2 by the residual rate (%) with respect to the transglutaminase activity when stored at 4°C.

As shown in Fig. 2, the liquid preparations of Examples 1-1 to 1-3 containing glycine at a concentration of 5 wt%, 10 wt% or 20 wt%, respectively, showed higher transglutaminase activity residual rates as compared to the control.

On the other hand, in the liquid preparation of Comparative Example 2 having a glycine content of 1 wt%, the residual activity rate of transglutaminase was similar to that of the control.

### (2) Proline content

Proline was added to and dissolved at 5 wt%, 10 wt%, 20 wt% and 30 wt% in 0.05 M phosphate buffer (pH=6.0) containing microorganism-derived transglutaminase (1,000U/g, "ActivaTG", Ajinomoto Co., Inc.) (17.6 wt%), and sodium benzoate (0.1 wt%). 0.05 M Phosphate buffer (pH=6.0) was used to make the total amount 100 wt% and the liquid preparations were used as Examples 2-1, 2-2, 2-3 and 2-4, respectively.

In addition, a liquid preparation prepared similarly with a proline content of 1 wt% was used as Comparative Example 3.

Respective liquid preparations of Examples 2-1 to 2-4 and Comparative Example 3 were stored at 4°C and 44°C for 24 hr, and the transglutaminase activity in the liquid preparation was measured by the above-mentioned hydroxamate method. A liquid preparation similarly produced without adding proline was used as a control and treated in the same manner.

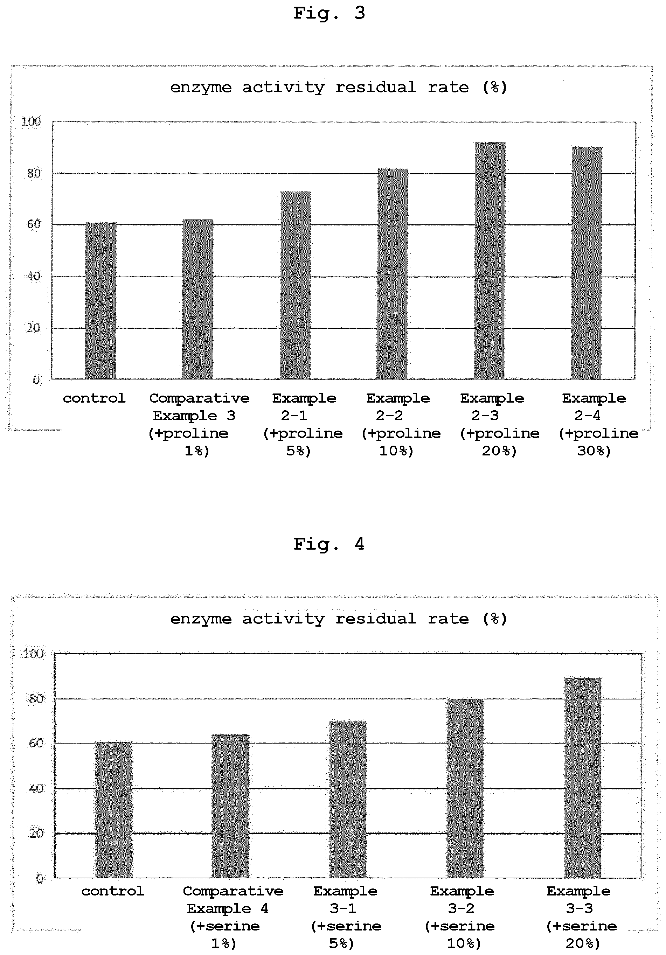

The transglutaminase activity when stored at 44°C is shown in Fig. 3 by the residual rate (%) with respect to the transglutaminase activity when stored at 4°C.

As shown in Fig. 3, the liquid preparations of Examples 2-1 to 2-4 containing proline at a concentration of 5 wt%, 10 wt%, 20 wt% or 30 wt%, respectively, showed higher transglutaminase activity residual rates as compared to the control. The transglutaminase activity residual rate in the liquid preparations of Examples 2-4 having a proline content of 30 wt% was not greatly different from the activity residual rate in the liquid preparations of Examples 2-3 having a proline content of 20 wt%, and it was suggested that the transglutaminase activity stabilizing effect reaches a plateau when the proline content exceeds 20 wt%.

On the other hand, in the liquid preparation of Comparative Example 3 having a proline content of 1 wt%, the residual activity rate of transglutaminase was similar to that of the control.

### (3) Serine content

Serine was added to and dissolved at 5 wt%, 10 wt% and 20 wt% in 0.05 M phosphate buffer (pH=6.0) containing microorganism-derived transglutaminase (1,000U/g, "ActivaTG", Ajinomoto Co., Inc.) (17.6 wt%), and sodium benzoate (0.1 wt%). 0.05 M Phosphate buffer (pH=6.0) was used to make the total amount 100 wt% and the liquid preparations were used as Examples 3-1, 3-2 and 3-3, respectively.

In addition, a liquid preparation prepared similarly with a serine content of 1 wt% was used as Comparative Example 4.

Respective liquid preparations of Examples 3-1 - 3-3 and Comparative Example 4 were stored at 4°C and 44°C for 24 hr, and the transglutaminase activity in the liquid preparation was measured by the above-mentioned hydroxamate method. A liquid preparation similarly produced without adding serine was used as a control and treated in the same manner.

The transglutaminase activity when stored at 44°C is shown in Fig. 4 by the residual rate (%) with respect to the transglutaminase activity when stored at 4°C.

As shown in Fig. 4, the liquid preparations of Examples 3-1 to 3-3 containing serine at a concentration of 5 wt%, 10 wt% or 20 wt%, respectively, showed higher transglutaminase activity residual rates as compared to the control.

On the other hand, in the liquid preparation of Comparative Example 4 having a serine content of 1 wt%, the residual activity rate of transglutaminase was similar to that of the control.

### (4) Sodium glutamate content

Sodium glutamate was added to and dissolved at 5 wt%, 10 wt%, 20 wt% and 30 wt% in 0.05 M phosphate buffer (pH=6.0) containing microorganism-derived transglutaminase (1,000U/g, "ActivaTG", Ajinomoto Co., Inc.) (17.6 wt%), and sodium benzoate (0.1 wt%). 0.05 M Phosphate buffer (pH=6.0) was used to make the total amount 100 wt% and the liquid preparations were used as Examples 4-1, 4-2, 4-3 and 4-4, respectively.

In addition, a liquid preparation prepared similarly with a sodium glutamate content of 1 wt% was used as Comparative Example 5.

Respective liquid preparations of Examples 4-1 - 4-4 and Comparative Example 5 were stored at 4°C and 44°C for 24 hr, and the transglutaminase activity in the liquid preparation was measured by the above-mentioned hydroxamate method. A liquid preparation similarly produced without adding sodium glutamate was used as a control and treated in the same manner.

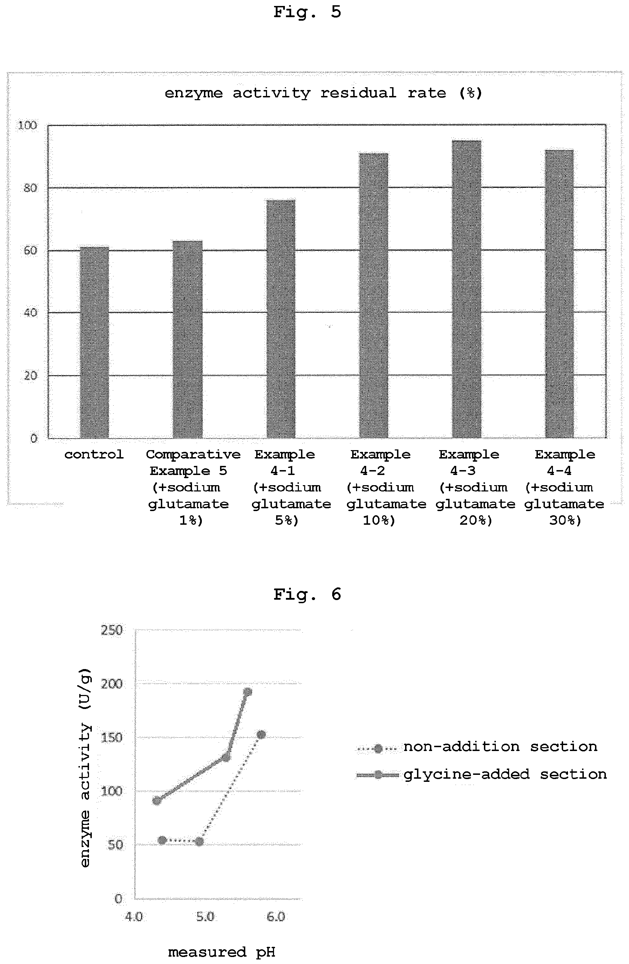

The transglutaminase activity when stored at 44°C is shown in Fig. 5 by the residual rate (%) with respect to the transglutaminase activity when stored at 4°C.

As shown in Fig. 5, the liquid preparations of Examples 4-1 to 4-4 containing sodium glutamate at a concentration of 5 wt%, 10 wt%, 20 wt% or 30 wt%, respectively, showed higher transglutaminase activity residual rates as compared to the control. The transglutaminase activity residual rate in the liquid preparations of Examples 4-4 having a sodium glutamate content of 30 wt% was not greatly different from the activity residual rate in the liquid preparations of Examples 4-3 having a sodium glutamate content of 20 wt%, and it was suggested that the transglutaminase activity stabilizing effect reaches a plateau when the sodium glutamate content exceeds 20 wt%.

On the other hand, in the liquid preparation of Comparative Example 5 having a sodium glutamate content of 1 wt%, the residual activity rate of transglutaminase was similar to that of the control.

The results of the above-mentioned Experimental Example 2 reveal that the transglutaminase stabilizing effect is found when the content of glycine, proline, serine and sodium glutamate in the liquid preparation is each not less than 5 wt%, and a more favorable stabilizing effect is obtained when the content of glycine, proline, serine and sodium glutamate is each not less than 10 wt%.

Also, it was suggested that the transglutaminase stabilizing effect reaches a plateau when the content of glycine, proline, serine and sodium glutamate each exceeds 20 wt%.

### [Experimental Example 3] Study of influence of pH of liquid preparation on stabilization of transglutaminase

As shown below, an influence of pH of a liquid preparation on the stabilization of transglutaminase was evaluated by an accelerated test by high temperature storage.

As a glycine-added section, a sample containing microorganism-derived transglutaminase (1,000U/g, "ActivaTG", Ajinomoto Co., Inc.) and glycine (10 wt%) was prepared according to the formulation of the liquid preparation shown in Table 2.

In each sample of the glycine-added section, glycine was replaced with each buffer to give each sample of the glycine non-addition section.

The samples in the glycine-added section and the glycine non-addition section were stored at 4°C and 44°C for 24 hr, and the transglutaminase activity in each liquid preparation was measured by the above-mentioned hydroxamate method. The results are shown in Fig. 6.

**[Table 2]**

| component/sample | content (wt%) | | |
|---|---|---|---|
| | pH=4 | pH=5 | pH=6 |
| transglutaminase (1,000U/g) | 17.6 | 17.6 | 17.6 |
| glycine | 10.0 | 10.0 | 10.0 |
| sodium benzoate | 0.1 | 0.1 | 0.1 |
| 0.05 M acetate buffer (pH=4.0) | 72.3 | | |
| 0.05 M acetate buffer (pH=5.0) | | 72.3 | |
| 0.05 M phosphate buffer (pH=6.0) | | | 72.3 |
| total | 100.0 | 100.0 | 100.0 |
| pH (Found) | 4.30 | 5.27 | 5.57 |

As shown in Fig. 6, in the liquid preparations with pH 4 - 6, it was found that the transglutaminase activity is high in samples added with glycine as compared to the sample free of glycine.

Also, it was found that the transglutaminase activity is particularly high in the samples containing glycine when the pH of the liquid preparation is 5 - 6.

From the above-mentioned results of Experimental Example 3, a good transglutaminase stabilizing effect was found when the pH of the preparation of the present invention was 4 - 6. It was suggested that the transglutaminase activity is maintained higher when the pH of the liquid preparation is 5 - 6.

### [Examples 6 - 8, Comparative Examples 6-1 to 8-2] Liquid preparation containing transglutaminase

Microorganism-derived transglutaminase (1,000U/g, "ActivaTG", Ajinomoto Co., Inc.) (17.6 wt%) and sodium acetate, sodium gluconate and sodium citrate (each 10 wt%) were dissolved in 0.05 M phosphate buffer (pH=6.0) (72.4 wt%) to give the liquid preparations of Examples 6 - 8.

In addition, the liquid preparations of Comparative Examples 6-1 to 8-2 were prepared in the same manner as in the above-mentioned each Example with the content of sodium acetate, sodium gluconate and sodium citrate as 1 wt% and 0.1 wt%.

### [Experimental Example 4] Evaluation of stabilizing effect of transglutaminase

The stability of transglutaminase in each liquid preparation of the above-mentioned Examples 6 - 8 and Comparative Examples 6-1 to 8-2 was evaluated by an accelerated test by high temperature storage as follows.

Respective liquid preparations of the above-mentioned Examples 6 - 8 and Comparative Examples 6-1 to 8-2 were stored at 4°C and 44°C for 24 hr, and the transglutaminase activity in the liquid preparation was measured by the above-mentioned hydroxamate method.

A liquid preparation obtained by dissolving only transglutaminase in a phosphate buffer was used as a control and treated in the same manner.

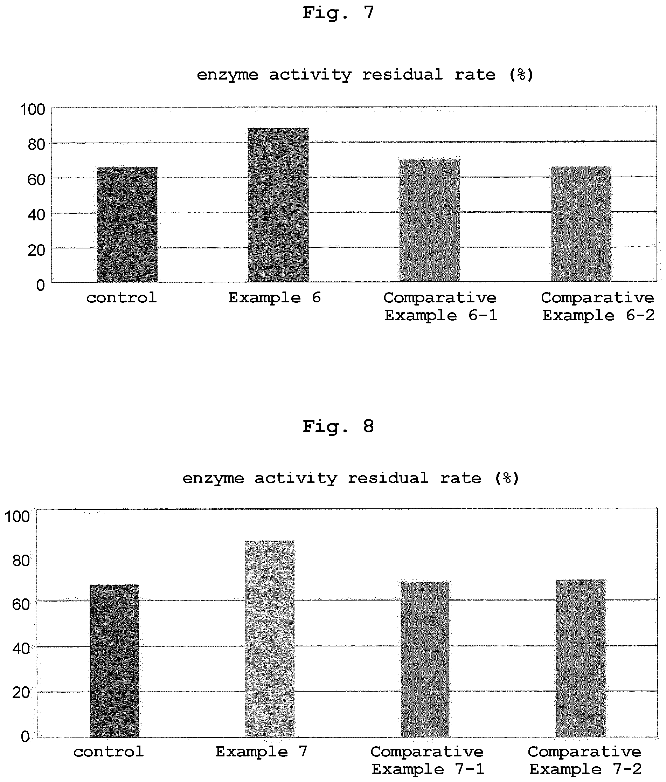

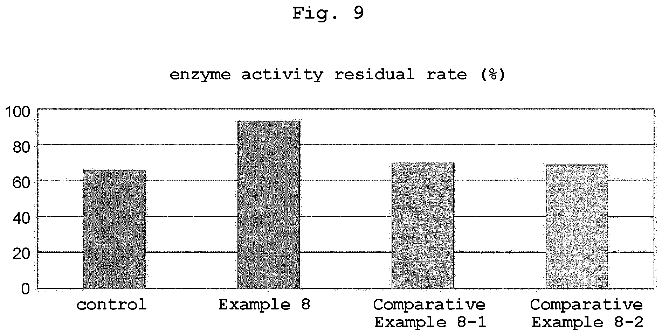

The stability of transglutaminase in each liquid preparation is shown in Figs. 7 - 9 by the residual rate (%) of the transglutaminase activity when stored at 44°C with respect to the transglutaminase activity when stored at 4°C.

As shown in Figs. 7 - 9, the liquid preparations of Examples 6 - 8 containing 10 wt% each of sodium acetate, sodium gluconate and sodium citrate showed clearly improved transglutaminase activity residual rate as compared to the control.

However, in the liquid preparations of Comparative Examples 6-1, 7-1 and 8-1 in which the content of sodium acetate, sodium gluconate and sodium citrate was 1 wt% for each, and the liquid preparations of Comparative Examples 6-2, 7-2 and 8-2 in which the content of sodium acetate, sodium gluconate and sodium citrate was 0.1 wt% for each, the transglutaminase activity residual rate was the same as that of the control.

From the above-mentioned results of Experimental Example 4, it was shown that sodium acetate, sodium gluconate and sodium citrate which are organic acid salts can also stabilize transglutaminase, and that transglutaminase is favorably stabilized when the content of the aforementioned organic acid salt in the liquid preparation is 10 wt%.

### [Industrial Applicability]

As described in detail above, according to the present invention, a liquid preparation of transglutaminase showing improved stability of transglutaminase can be provided.

The liquid preparation of the present invention does not require dissolution of transglutaminase in a solvent when in use, is highly convenient, and is associated with less risk of microorganism contamination and the like. It is suitable for use in the field of food.

In addition, according to the present invention, a food containing a food material such as meat and the like on which transglutaminase having improved stability has acted can be provided by adding the above-mentioned liquid preparation of the present invention.

This application is based on a patent application No. 2018-057185 filed in Japan, the contents of which are incorporated in full herein.

## Claims

1. A liquid preparation comprising transglutaminase, and one or more kinds selected from the group consisting of glycine, proline, serine, a glutamic acid salt, an aspartic acid salt and an organic acid salt, wherein a total content of said one or more kinds selected from the group consisting of glycine, proline, serine, a glutamic acid salt, an aspartic acid salt and an organic acid salt is not less than 5 wt%.

2. The liquid preparation according to claim 1, wherein the transglutaminase is derived from a microorganism.

3. The liquid preparation according to claim 1 or 2, wherein the total content of said one or more kinds selected from the group consisting of glycine, proline, serine, a glutamic acid salt, an aspartic acid salt and an organic acid salt is not less than 10 wt%.

4. The liquid preparation according to any one of claims 1 to 3, wherein the organic acid salt is one or more kinds selected from the group consisting of an acetic acid salt, a citric acid salt and a gluconic acid salt.

5. The liquid preparation according to any one of claims 1 to 4, wherein the glutamic acid salt, aspartic acid salt and organic acid salt are each a sodium salt.

6. The liquid preparation according to any one of claims 1 to 5, wherein the liquid preparation has pH 4 - 7.

7. A liquid preparation comprising transglutaminase, and one or more kinds selected from the group consisting of glycine, proline, serine, a glutamic acid salt, an aspartic acid salt and an organic acid salt, wherein a total content of said one or more kinds selected from the group consisting of glycine, proline, serine, a glutamic acid salt, an aspartic acid salt and an organic acid salt is not less than 2 wt%.

8. A method for producing a liquid preparation comprising dissolving transglutaminase, and one or more kinds selected from the group consisting of glycine, proline, serine, a glutamic acid salt, an aspartic acid salt and an organic acid salt by adding them to a solvent, wherein a total content of said one or more kinds selected from the group consisting of glycine, proline, serine, a glutamic acid salt, an aspartic acid salt and an organic acid salt is not less than 5 wt%.

9. The production method according to claim 8, wherein the transglutaminase is derived from a microorganism.

10. The production method according to claim 8 or 9, wherein the total content of said one or more kinds selected from the group consisting of glycine, proline, serine, a glutamic acid salt, an aspartic acid salt and an organic acid salt is not less than 10 wt%.

11. The production method according to any one of claims 8 to 10, wherein the organic acid salt is one or more kinds selected from the group consisting of an acetic acid salt, a citric acid salt and a gluconic acid salt.

12. The production method according to any one of claims 8 to 11, wherein the glutamic acid salt, aspartic acid salt and organic acid salt are each a sodium salt.

13. The production method according to any one of claims 8 to 12, comprising setting the pH of the liquid preparation to 4 - 7.

14. A method for producing a liquid preparation comprising dissolving transglutaminase, and one or more kinds selected from the group consisting of glycine, proline, serine, a glutamic acid salt, an aspartic acid salt and an organic acid salt by adding them to a solvent, wherein a total content of said one or more kinds selected from the group consisting of glycine, proline, serine, a glutamic acid salt, an aspartic acid salt and an organic acid salt is not less than 2 wt%.

15. A method for stabilizing transglutaminase in a liquid preparation, comprising adding one or more kinds selected from the group consisting of glycine, proline, serine, a glutamic acid salt, an aspartic acid salt and an organic acid salt to a liquid preparation comprising transglutaminase such that a total content of these is not less than 5 wt%.

16. The stabilizing method according to claim 15, wherein the transglutaminase is derived from a microorganism.

17. The stabilizing method according to claim 15 or 16, wherein the total content of said one or more kinds selected from the group consisting of glycine, proline, serine, a glutamic acid salt, an aspartic acid salt and an organic acid salt is not less than 10 wt%.

18. The stabilizing method according to any one of claims 15 to 17, wherein the organic acid salt is one or more kinds selected from the group consisting of an acetic acid salt, a citric acid salt and a gluconic acid salt.

19. The stabilizing method according to any one of claims 15 to 18, wherein the glutamic acid salt, aspartic acid salt and organic acid salt are each a sodium salt.

20. The stabilizing method according to any one of claims 15 to 19, comprising setting the pH of the liquid preparation to 4 - 7.

21. A method for stabilizing transglutaminase in a liquid preparation, comprising adding one or more kinds selected from the group consisting of glycine, proline, serine, a glutamic acid salt, an aspartic acid salt and an organic acid salt to a liquid preparation comprising transglutaminase such that a total content of these is not less than 2 wt%.

22. A food comprising the liquid preparation according to any one of claims 1 to 6.

23. A food comprising the liquid preparation according to claim 7.
